Europäisches Patentamt

European Patent Office

Office européen des brevets

(11) Publication number: **0 295 349**
**A2**

## (12) EUROPEAN PATENT APPLICATION

(21) Application number: 87309561.6

(22) Date of filing: 29.10.87

(51) Int. Cl.⁴: **A22B 5/00** , //G01N33/12

(30) Priority: 19.06.87 US 65065

(43) Date of publication of application:
21.12.88 Bulletin 88/51

(84) Designated Contracting States:
AT BE CH DE ES FR GB GR IT LI LU NL SE

(71) Applicant: IDETEK, INC:
1057 Sneath Lane
San Bruno California 94066(US)

(72) Inventor: Singh, Prithipal
25627 Elena Road
Los Altos Hills California 94022(US)
Inventor: Oliver, Diane G.
57 Bridge Road
Ross California 94957(US)
Inventor: Allison, David E.
830 Bryant Street
Palo Alto California 94301(US)

(74) Representative: Harrison, David Christopher
et al
MEWBURN ELLIS & CO 2/3 Cursitor Street
London EC4A 1BQ(GB)

(54) **Automated slaughterhouse screening.**

(57) A method for use in a modern slaughterhouse operation for testing carcasses to be processed for one or more characteristics indicative of a carcass containing harmful, questionable or undesirable matter is substantially automated using unique bar code labels (25) on test tube racks (17), test tubes (16) and microplates (40) along with a computer to record and display individual test results in an organized fashion. The computer reads optical density values from a spectrophotometer and calculates test results for the samples based upon these values. Positive test results are displayed prominently indicating that the corresponding carcass may contain questionable matter. The testing is done using an immunoassay which would be effective for the detection of trichinosis in swine, or a standard ELISA test effective for detecting a level of sulfamethazine in pigs indicative of the use of antibiotics. Test results are available well before the dismemberment and processing of a carcass, such that if a positive test result is encountered the corresponding carcass may be removed from the routine processing procedure.

EP 0 295 349 A2

FIG.8

## AUTOMATED SLAUGHTERHOUSE SCREENING

The present invention relates generally to slaughterhouse operations, and more particularly to techniques used in slaughterhouses to prevent the processing and distribution of potentially harmful edible products originating from an infected or contaminated animal carcass which may contain harmful substances.

One of the main objectives of a modern slaughterhouse operation is to maximize the rate at which carcasses are processed while still insuring a high standard of quality for the resulting edible products. Through the utilization of modern technology, slaughterhouse operations have enjoyed the opportunity to continually increase the rate at which carcasses are processed. Many useful devices, such as electric prods for stunning the animals and conveyor belts for transporting the carcasses, have been incorporated into nearly all stages of the processing operation in order to assist the workers in increasing productivity. Although many useful developments have been made in the area of increasing productivity, very little progress has been made in the area of testing the carcasses for possible infectious or harmful matter.

Currently, in modern slaughterhouse operations if there is any testing done for infectious or harmful substances, it is usually limited to a mere visual inspection of the carcasses. These visual inspections are only capable of detecting carcasses which have been already corrupted by infection or disease to the point where there is visible damage. Needless to say, an infected carcass showing no signs of physical deterioration will be routinely processed with the other carcasses, contaminating the resulting edible products.

Various other testing techniques have been available, but due to one or more limitations inherent in these techniques, they are impractical for use in the slaughterhouse environment. It is desirable, although heretofore impractical, to insure that all the products produced by the slaughterhouse are free from harmful infectious or diseased matter. To accomplish this desired goal, every carcass to be processed in the slaughterhouse should be individually tested for any characteristics that indicate the presence of harmful or infectious matter.

One of the limitations inherent in some prior art testing techniques is the inordinate amount of time it takes to yield results. The length of time to yield results in these techniques is unacceptable in that by the time the results are known the carcasses in the slaughterhouse have already been dismembered and processed, and the opportunity to detain carcasses with positive results has passed. Many of the prior art testing techniques fall into the category of microbiological testing procedures. Typically, microbiological testing involves taking a tissue sample from a carcass and placing it on a very specific media in a petri dish. This media is known to be a media in which the particular bacteria or other microorganism of interest will flourish. After an incubation period, usually on the order of several days, some of the sample is removed and a reagent is added. If a pre-established change, such as agglutination occurs then the sample is positive, otherwise it is negative. Clearly, the results of such a test cannot practically be done on a large scale basis as would be required in a slaughterhouse operation, and even if this were possible, the results of such a test would be available only well after the carcasses have been dismembered and processed. Additionally, the preparation of the specific culture media is relatively expensive, particularly if used on a large scale basis as would be required in a slaughterhouse environment.

Another of the prior art testing techniques is the so-called "digestion" method. This method is used extensively for the testing of trichinosis, an intestinal and tissue infection caused by the nematode Trichinella spiralis. This rather crude technique involves incubating a tissue sample (usually about 5 gm) with a pepsin solution under acidic conditions, mixing and straining the resulting solution, then viewing the resultant strained matter under a low-power microscope. When viewing with the microscope, one looks for the actual worms or nematodes amongst similar matter from the sample. A test such as this is very demanding for the trained observer and requires a tremendous amount of concentration to accurately read results. If at all attempted to be carried out in a practical fashion, the test will require at least two individuals; one to prepare the samples for viewing, and another to view the prepared samples under a microscope. This rather arduous technique yields subjective results which are dependent upon the particular observer's concentration and perception.

Through the use of more recent methods developed for determining a component of the antigen-antibody reaction, such as disclosed in the Schuurs et al. U.S. patent Nos. 3,654,090, 3,791,932 and 3,850,752, enzyme-linked immunoassay techniques have proven useful in detecting the presence of a characteristic associated with substances of interest. However, these techniques have also been limited to the laboratory environment for reasons of convenience and as such, could only be used for infrequent testing of random samples from a slaughterhouse operation.

## Summary of the Invention

It is an object of the present invention to provide a method which may be used in a modern slaughterhouse operation for testing each, or some of the carcasses to be processed, for one or more characteristics which indicate a carcass may contain matter which, if processed, would result in the production of potentially harmful or undesirable edible products.

It is a related object of the present invention to provide such a testing method which is substantially automatic, thereby effectively eliminating most sources of human error which would tend to alter or produce misleading results of tests conducted.

It is another object of the present invention to provide such a testing method which can yield test results for any particular carcass well before the carcass is dismembered, such that if found to contain undesirable matter, the carcass may be detained or removed from the routine processing procedures.

Further in that regard, it is an object of the present invention to provide such a testing method that relates each individual test result to the particular carcass from which the result was derived so that in the event a particular result is positive (i.e. indicates a harmful or infected carcass has been encountered), the corresponding carcass may be identified, located and prevented from routine processing.

It is an aspect of the present invention to provide such a testing method that may easily be modified to produce results for several characteristics, each of which may indicate a different infectious or harmful matter present in the associated carcass.

According to that aspect of the invention, it is still another aspect to provide such a testing method in which the testing for several indicative characteristics is carried out substantially simultaneously.

It is yet another aspect of the present invention to provide such a testing method in which a carcass is tested for characteristics indicative of possible chemical or physical abuse of the animal such as by growth promoting substances or antibiotics.

The above objects are realized in the present invention which provides an accurate, efficient and substantially automated method for use in a slaughterhouse operation which is capable of testing each carcass to be processed in the slaughterhouse operation for several characteristics indicative of the carcass being unsuitable for routine processing. In accordance with the method of the present invention, the test results for any particular carcass are available well before the carcass is to be dismembered such that if a positive test is encountered, the carcass may be removed from the routine processing operation.

The method provides an organized approach for relating the results of each individual test to the particular carcass from which the test result was derived. This provides an efficient means for determining and locating a particular carcass which produces positive test results, so that this carcass of concern may be removed from the routine processing operation in order to prevent contamination and production of undesirable edible products.

The results of the testing method are easily and routinely derived through an automated procedure thereby establishing a high degree of reliability and effectively eliminating human error from the procedure which would be present in some of the rather subjective prior art testing techniques.

## Brief Description of the Drawings

Other objects and advantages of the present invention will be apparent from the following detailed description and upon reference to the drawings, in which:

Fig. 1 is a perspective view of a slaughterhouse operation where the method of the present invention is being used;

Fig. 2 is a perspective view of a test tube with an attached bar code label used for collecting a fluid sample from a carcass;

Fig. 3 is a perspective view of a test tube rack used to collect samples according to the present invention;

Fig. 4 is a perspective view depicting a microplate to be used in conjunction with the test tube rack of Fig. 3;

Fig. 5 is a perspective view of a robotic sample processor used to prepare assays for the present invention;

Fig. 6 is a sample of a computer input and display screen used to enter identifying information for each plate/rack combination;

Fig. 7 is a sample of a computer input and display screen for entering sample codes for each plate/rack combination;

3

Fig. 8 is a flow diagram depicting the various steps involved in the method according to the present invention;

Fig. 9 is an optical density value chart as would be displayed on a video monitor connected to a computer used with the present invention;

Fig. 10 is a chart displaying the values of the immunoassay determined from samples, such a chart relates to the chart of Fig. 9 and would also be displayed on a video monitor connected to the computer.

While the invention is susceptible to various modifications and alternative forms, an exemplary embodiment thereof is shown by way of example in the drawings and will herein be described in detail. It should be understood, however, that it is not intended to limit the invention to the particular embodiment disclosed, but on the contrary, the intention is to cover all modifications, equivalents, and alternatives falling within the spirit and scope of the invention as defined by the appended claims.

The method of the present invention is accomplished through the use of apparatus including a microcomputer operated in combination with immunoassay apparatus. The microcomputer provides means for organization of the individual samples and test results, analysis of data produced from the immunoassay apparatus and display of information relating to the test results. The immunoassay apparatus provides means for testing the non-solid tissue samples from the carcasses for the presence of antibodies or antigens indicative of a carcass containing undesirable, harmful or questionable matter. The results of an immunoassay test are available typically within about 30-120 minutes from the time a sample is available. The immunoassay apparatus is automated and highly efficient, capable of producing test results for many samples simultaneously. This highly automated feature of the apparatus allows it to complete the testing procedures with a minimum of human intervention, thereby effectively eliminating the introduction of most sources of human error into the system.

In the preferred embodiment of the invention the non-solid tissue samples are blood samples, which are easily collectible just after a stunned or shocked animal enters the slaughterhouse and a major artery is severed in the the slaughtering process. The samples are gathered in test tubes which are organized and stored in test tube racks. In order to uniquely identify the liquid sample procured from each carcass, a unique bar code label with a visible number is attached to each test which is to be used to collect a sample. Associated with each test tube rack is a microplate used for carrying out the assays. The number and arrangement of the wells in each microplate correspond to the number and arrangement of positions available in the test tube rack. In order to uniquely identify each test tube rack with its associated microplate, and vice versa, there is provided a dual bar code label for each plate/rack combination. One half of the dual bar code label is placed on the microplate, and the other half is placed on the associated test tube rack, thereby providing a unique identifier for each plate/rack combination. In order to provide an additional degree of automatization, the bar code labels are preferably of the type that are easily read electronically as with an infrared bar code reader wand.

The test tube rack is supplied with samples in a sequential manner. To achieve a high degree of reliability, the assays may be carried out in duplicate on the microplate; each test tube sample, in this case, will correspond to two wells of the microplate, and likewise, two resulting assay determinations for each sample.

According to another desirable feature for providing an automated method, the assay preparation is carried out automatically using a robotic sample processor to transfer a small amount of a blood, plasma, serum or urine sample from a test tube to the corresponding well or wells of the microplate. The robotic sample processor insures that the proper amount of sample is distributed to the proper locations on the microplate. The wells of the microplates have previously been prepared with a coating of an antigen or antibody necessary to react with the sample. The wells of the microplate are further processed with steps such as washing, incubating, and adding conjugates and reagents to produce the proper assay sites in the wells. The microplate is then inserted into a spectrophotometer, preferably a vertical light path spectrophotometer, and the optical densities of each of the wells is determined.

The values of the optical density determination are automatically transferred to the microcomputer via an interface cable, such as an RS232C interface connector. Through the use of a specially designed computer program, the optical densities are processed resulting in the calculation of the test results for each sample. The optical density values and the calculated results are conveniently displayed on a monitor. Any values which are indicative of a sample testing positive (i.e. the corresponding carcass contains harmful matter), are displayed with prominence by using video characteristics such as highlighting and flashing. The enhanced video characteristics are used to catch the attention of the person reading the display, so that he will be alerted that a questionable sample has been encountered. In addition to being available for display, the optical density and immunoassay values may be stored for future reference. Options are also available for using these values to create a hardcopy table for reference purposes. The microcomputer may also be

4

useful to track down in the slaughterhouse a carcass which has yielded a positive test result. This could be done by using the recorded sample data in conjunction with information regarding the slaughterhouse in order to produce a location or area in which the carcass may reside so it may be detained or removed from the routine processing operation. Further manipulations of the data are possible for creating statistical determinations for several tests conducted.

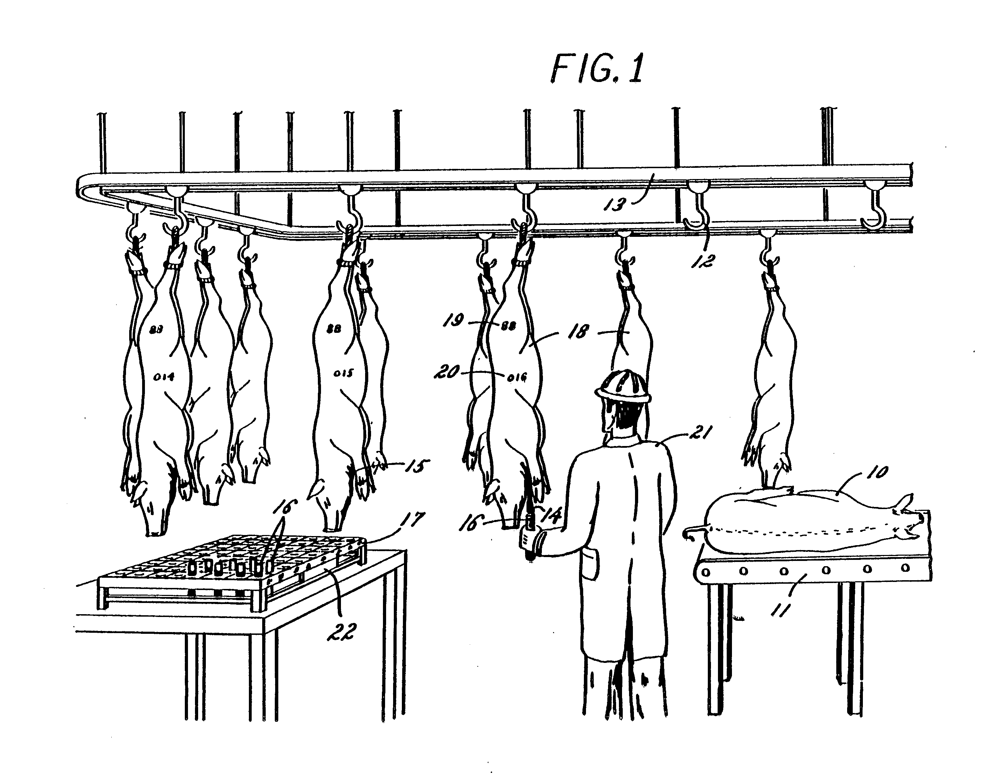

Turning now to Fig. 1, there is shown a perspective view of a modern high-speed slaughterhouse operation, which in this particular example, is prepared for the processing of swines. The stunned animals 10 enter the slaughterhouse via a conveyor belt 11 or chute at rates of typically three to twenty animals per minute. Upon entering the slaughterhouse the animals are suspended on shackles 12 which in turn are supported and guided on racks 13. At this time, a major artery of the animal is severed, causing the profuse drainage of blood 14 from the resulting laceration 15. Alternatively, the animals may be killed in their horizontal positions. According to one important aspect of the invention, a blood sample from each carcass, or predetermined number or group of carcasses, is collected in test tubes 16 from the draining stream of blood. These test tubes 16 are preferably arranged on a sequential basis in rows of a test tube rack 17.

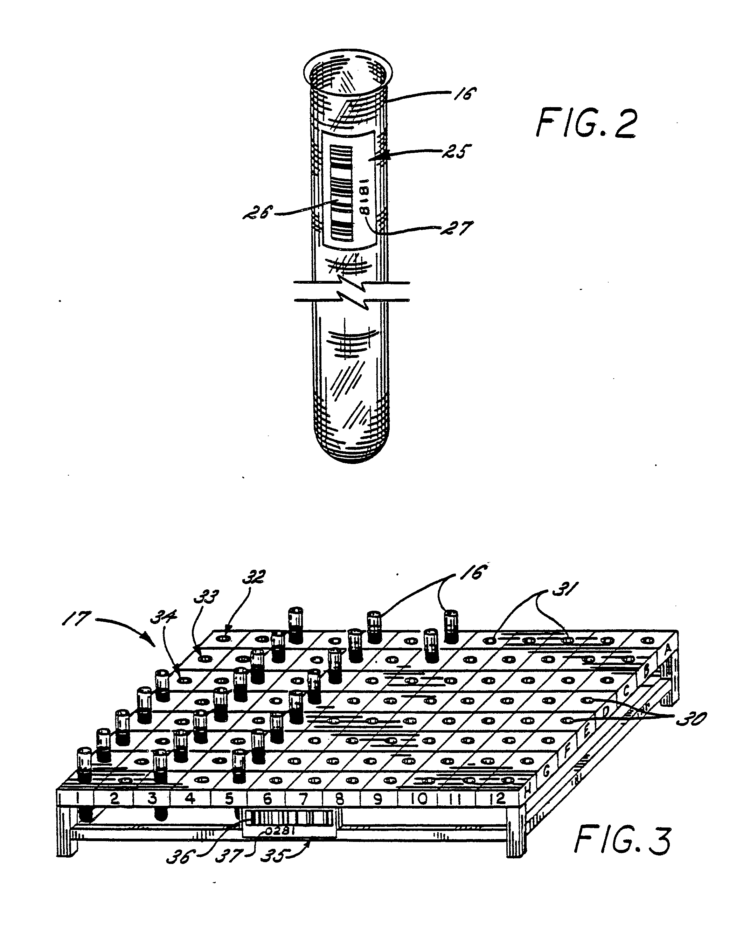

Turning for a moment to Fig. 2, there is shown a test tube 16 used to collect a non-solid tissue sample from a carcass. The non-solid tissue samples may take many forms including blood, plasma, serum or urine. As can be seen, a bar code label 25 is attached to the test tube 16 and includes a bar code section 26 which is readable via bar code reader wand, and a numeric section 27 containing a number easily readable by a human such as a slaughterhouse worker or a laboratory worker. The number encoded in the bar code section 26 of the label 25 is the same as the number in the numeric section 27. As will be described in more detail later, an area 22 is located on the test tube rack for a bar code label which will be attached in order to provide a unique and convenient means for positive identification of the rack 17. As can be seen, the carcasses 18 are tatooed or stamped with two identifying labels. The first label 19 is typically a farm designation, while the second label 20 is a unique, sequential numeric identifier. The numeric identifier 20 which is placed on a particular carcass is identical to the number located on the bar code label 25 used to collect a sample from that carcass. For example, in reference to Fig. 1, the carcass from which the worker 21 is collecting a sample is labeled with the same unique numeric identifier as found on the test tube, this being '016' in the example.

Turning now to Fig. 3, there is shown a more detailed perspective view of the test tube rack 17 used to collect the non-solid tissue samples from the carcasses. This particular rack 17 has 8 rows 30 labeled A through H and 12 columns 31 numbered 1 through 12, and is capable of holding 96 test tubes 16. Upon preparation of the samples from the test tube rack for the immunoassay, one half of a unique dual bar code label 35 is taken from a microplate and attached to the test tube rack 17 thereby providing a unique identification for each test tube rack and plate combination. Similar to the bar code label used for a test tube, the test tube rack bar code label 35 consists of an encoded bar code section 36 and a numeric section 37, both of which correspond exactly to the same sections of the associated microplate bar code label. As mentioned previously, only every other column 31 of the rack 17 is used so as to allow for duplicate testing of the samples on an associated microplate. Typically, three positions in the test tube rack are reserved for the negative reference standard, positive reference standard and the positive control used in the particular test procedure. Typically, these positions are designated as A1 for the negative reference standard position 32, B1 for the positive reference standard position 33 and C1 for the positive control position 34. These positions on the test tube rack 17 are left unoccupied; the proper substances will be placed in the corresponding positions on the microplate at a later stage.

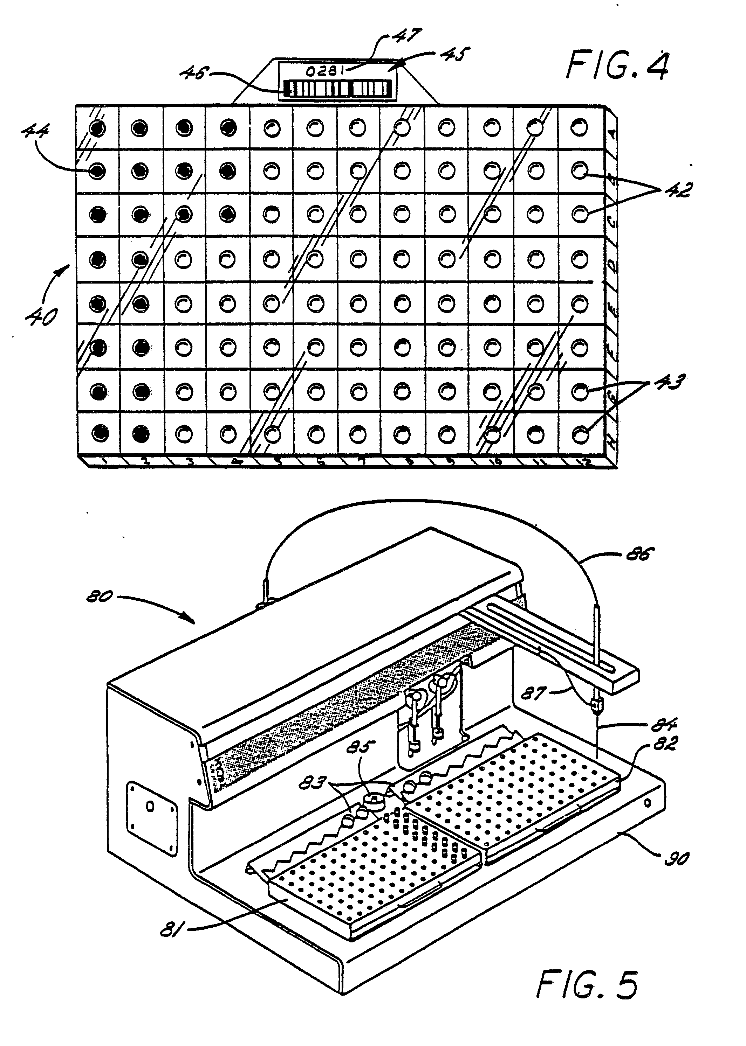

Turning now to Fig. 4, there is shown a corresponding microplate 40 used in the preparation and carrying out of the assays for the samples. The microplate 40 uses a bar code label 45 which is identical to the bar code label placed on the corresponding test tube rack, and similarly includes a corresponding numeric section 47 and bar code section 46. As with the positions of the test tube rack, the microplate has 8 rows 41 and 12 columns 42 of corresponding wells 43 which are similarly designated. According to an important object of the present embodiment, the assays may be carried out in duplicate, as will be described in the present embodiment. One blood sample 44 from a single test tube is placed in two wells 43 of the microplate. For example, from the test tube located in position D1 of the test tube rack 17 there will be placed blood samples into the two corresponding wells D1 and D2 of the microplate 40. Accordingly, these two samplings will each yield results which can be combined or compared against each other for accuracy. Preferably, if the differences in the results in the duplicate samples are greater than a certain predetermined limit, then the particular sample in question may be tested again to insure accuracy.

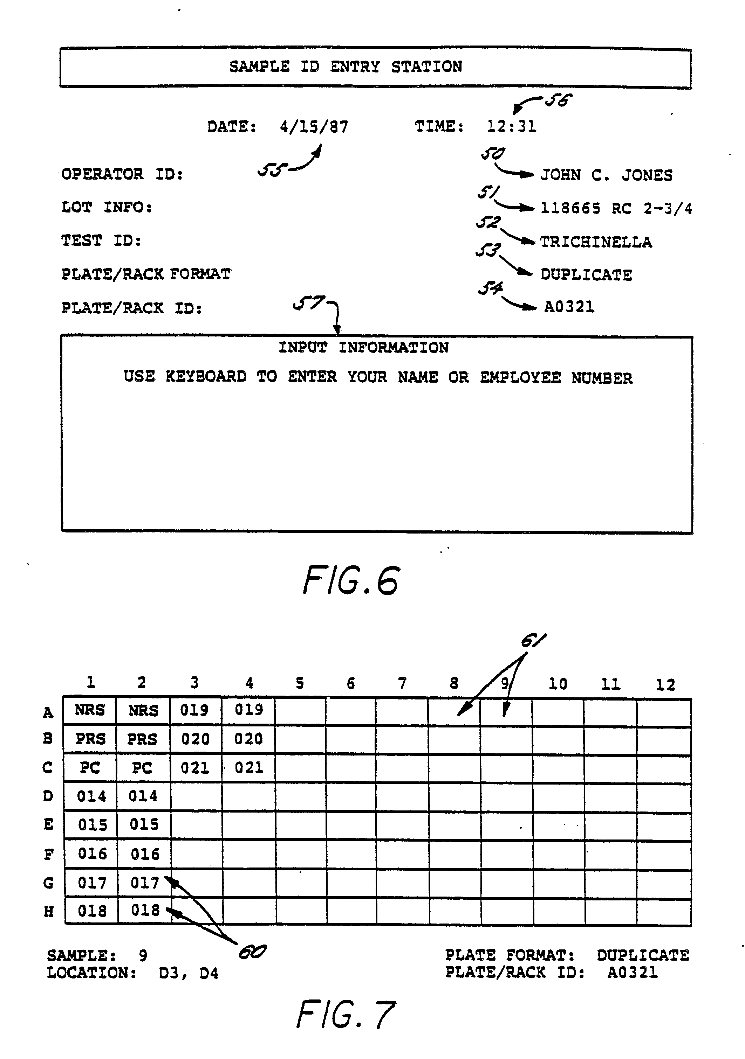

Referring now to Figs. 6 and 7, there is shown the preferred method for organizing the racks and plates and entering their format into a computer for storage, whereby such information may be retrieved for reference at later stages. As can be seen in Fig. 6, this display screen is used to enter information identifying the particular plate. The OPERATOR ID 50 is entered, identifying the person running the test. The LOT INFORMATION 51 is entered, and may contain information concerning the particular farm the animals came from or the reagent lot identification to be used for the assays. The TEST ID 52 is entered next, and contains information about the type of test to be conducted or the particular disease being tested for. The PLATE/RACK FORMAT 53 is entered next and is either free, single or duplicate format. In the single format, each well contains a different sample. Such a format could be used for particular tests which are known to have an inherent high degree of accuracy and therefore duplicate testing would be unnecessary. On the other hand, a free plate format may be used to increase the number of tests conducted for each sample, such as providing for testing in triplicate if desired.

The next item entered on this display screen is the PLATE/RACK ID 54 code. As mentioned previously, this code is attached to each microplate and its corresponding test tube rack using a dual bar code label. The bar code label on the microplate is exactly the same as the bar code label on the corresponding rack, thereby providing a unique identifier for a particular plate/rack combination which may easily and automatically be read using a bar code reader wand, or by viewing the numeric section. In addition to displaying the current date 55 and time 56 on the display screen, an area 57 is reserved for providing information and comments to assist a user during the input process.

In the next display screen shown in Fig. 7, numeric codes 60 are entered from the bar code label on each test tube sample. The user can move to various cells 61 on this screen by using the arrow keys on the keyboard or other designated keys if arrow keys are not available. These numeric codes 60 will also be used in association with the associated microplate whereby corresponding numeric codes 60 are assigned to particular wells of the plate. As can be seen, this example uses the duplicate testing procedure in which one numeric code is assigned to two well locations where tests of the same sample will be conducted. Three pairs of wells in the upper left portion of the plate are automatically reserved for the negative reference standard, positive reference standard and positive control and are assigned codes of NRS, PRS and PC respectively. The numeric codes entered correspond to the codes which have been placed on the carcasses. For example, if a test tube from position D1 with a bar code label number of '014' is used to collect a sample from a particular carcass, this carcass is then labeled with the number '014' while the code of '014' will be entered in position D1 of the present input screen. If the format of the plate is designated as duplicate format, then the computer automatically enters this code '014' in position D2 as well. Preferably, the test tube codes are ordered sequentially such that the plate/rack position codes may also be entered sequentially.

In accordance with the object of providing a highly reliable method of testing, each of the numeric codes 60 entered through the input screen of Fig. 5 are checked against all previous codes entered to insure that the current code being entered is unique. If the code being entered is found not to be unique, the computer will alert the user with a warning message and will not allow the code to be entered. In another embodiment, the user could be prompted to enter only the first numeric code 60 of a sequentially arranged plate/rack. After this first code is entered, then the computer automatically enters all the following sequential codes 60 for the entire plate/rack.

After entering the plate/rack format and all the codes associated therewith, this information is stored on a magnetic storage medium such as a floppy disk, hard disk or tape. This plate/rack information will be retrieved and used at a later stage when the assay test has been completed and the results for the various samples are available.

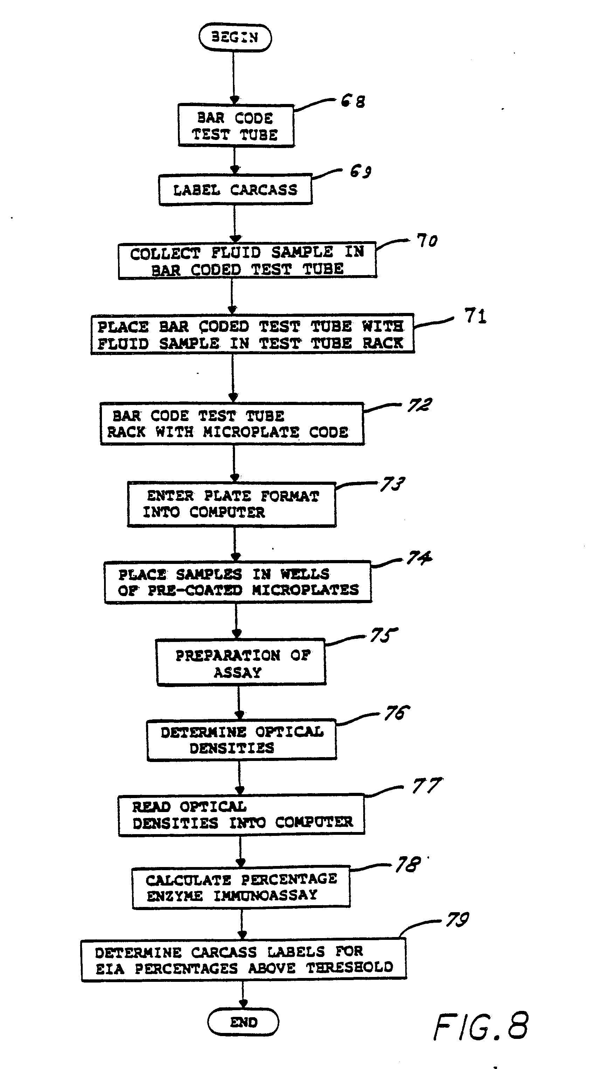

Turning now to Fig. 8, there is shown a flow diagram depicting the various steps involved in the testing procedure. Starting with step 68, a test tube which is to be used to collect a sample is uniquely identified by placing a bar code label on its outer surface. Next in step 69, the carcass is uniquely labeled with the same code as on the test tube, preferably by having a numeric code firmly tatooed to the carcass. The non-solid tissue sample is then collected in step 70 in the test tube with the same numeric code on its label as now exists on the corresponding carcass. Next in step 71, the bar coded test tube with the fluid sample is placed in a test tube rack. In step 72 the test tube rack containing bar coded test tubes with fluid samples from the carcasses is bar coded with one-half of a dual bar code label from a microplate which is placed on the test tube rack, thereby uniquely identifying the plate/rack combination. Next in step 73, the microplate format, associated codes and identifying information are entered into the computer. In step 74 samples are placed in wells of the microplate which has been pre-coated with an antigen or antibody of interest. Preferably step 74 is done automatically by using a robotic sample processor to transfer an aliquot of sample from a test tube to the proper wells in the microplate. The assay is properly prepared in the microplate in step 75. This preparation typically includes incubating and washing the microplate several times, and adding conjugate and substrate substances in order to produce the desired reaction.

Next in step 76, the optical densities of the prepared samples in the wells are determined. This is done preferably by using a vertical light path spectrophotometer. In step 77 the optical densities of the samples are read into the computer. In step 78, the optical density information is then used in conjunction with the microplate format information previously stored, to determine the percentage enzyme immunoassay for each sample. Next in step 79, it is determined which samples contain EIA percentages which exceed a certain predetermined threshold. Upon finding these samples, the carcass labels for these particular samples are determined by using the computer to find the particular sample code, which in turn has been tatooed on the carcass. The carcass with this code has yielded test results indicating it possibly contains undesirable matter and therefore should be located in the slaughterhouse and removed from the routine processing operation.

Turning now to Fig. 5, there is shown a robotic sample processor 80 used to transfer samples from a test tube to the wells of an associated microplate. Placed upon the table 90 of the device is one rack 81 of test tubes containing blood samples and one pre-coated microplate 82 to which samples from the test tubes will be transferred to its wells. As can be seen, the sample processor 80 contains reagent racks 83 for holding the reagents used to prepare the assay. These reagent racks 83 may also be used to hold the positive reference standard, negative reference standard and positive control substances. In order to cleanse the sampling tip 84 between the transfer of different samples, there is provided a washing station 85 which typically contains distilled water which is forced in and around the sampling tip 84, rinsing it thoroughly and preventing contamination of the samples. Pipetting tubing 86 is attached to the sampling tip 84 to extract and dispense the liquid samples. In order to insure that enough sample is present in the test tube, a liquid detector cable 87 monitors the capacitance of the sampling tip 84 for the presence of a liquid.

The arm 88 of the device allows for three dimensional movement of the tip 84 to any location on the table 90. Typically, the movement of the arm 88 is controlled via a series of stepper motors and as such is extremely accurate. Usually the robotic sample processor 80 is programmable for a series of steps which are necessary for the assay preparation. Dependent upon the particular assay being prepared, the steps include the transfer of samples and adding reagents and conjugates. Through programming, these steps may be precisely timed as is often desirable during the preparation of assays.

As mentioned previously, a vertical light path spectrophotometer is connected to the computer via a connection cable, such as an RS232C interface connector. The spectrophotometer provides an efficient means for simultaneously determining the optical densities of all the samples on the microplate. A connection cable is provided to quickly and accurately supply the computer with data corresponding to the optical densities of the samples when they have been determined.

Upon the computer determining that optical density information is available from the spectrophotometer, the user is prompted at the terminal to enter the plate ID code corresponding to the data. After this code is entered, the computer retrieves from magnetic medium the previously stored plate format and information. This information is then used to create a display on the computer screen indicating the optical density of each sample and other useful information which is determined using the optical density values. The optical densities of the samples are often compared to the optical density values of the positive reference standard, negative reference standard and positive control to determine a characteristic value of the sample, such as a percentage enzyme immunoassay value. If this characteristic value falls outside of a predetermined "safe" range, then the user is alerted of this fact on the video display screen of the computer.

Additional information alerting the user may also be be displayed at the screen. For example, if the optical density values of a sample used in duplicate testing differ significantly, then the user should be alerted of this situation. The test for such a sample may be repeated to insure its accuracy.

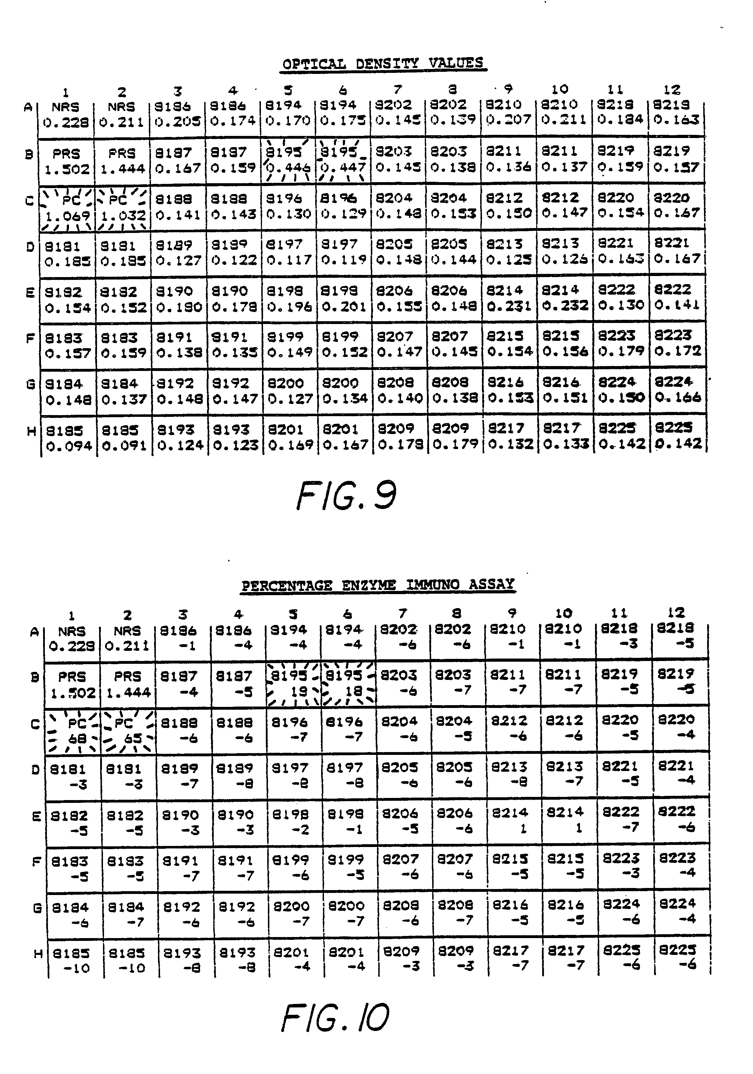

Turning now to Fig. 9, there is shown an example of a display screen used to display the optical density values for the samples. As can be seen, this display resembles the format of the microplate from which the values were derived. The display screen contains 96 different cells, each containing information about a particular sample. Preferably, each cell contains the sample position code and the corresponding optical density value. The contents of a cell that contains values that fall outside of a predetermined range may flash or use other enhanced video characteristics. In the example, the cells at locations B5 and B6 flash, indicating an optical density value above a predetermined upper bound of '0.4'. The values of the positive control PC also flash, indicating an optical density value above the threshold of '0.4'.

Referring now to Fig. 10, there is shown an example of a display screen which would be useful when the present invention is used for the detection of trichinosis. The various cells of this display contain the sample numbers along with their respective values of the enzyme immunoassay percentage. This EIA percentage is an indication of the presence of trichinosis in the carcass, and is calculated using the optical density values. One of the methods for this calculation is:

$$\%EIA = \frac{OD_{sample} - OD_{NRS}}{OD_{PRS} - OD_{NRS}} \times 100$$

As can be seen in Fig. 10 the values of the positive control PC (cells B1 and B2) and sample number 8195 (cells B5 and B6) flash. This flashing alerts the user, indicating that these samples have an EIA percentage which is greater than the predetermined threshold for this example.

An enzyme-linked immunoassay may be used for the detection of trichinosis by using antigen coated microplates for the assay preparation. The test is started by programming the robotic sample processor to pipet 10 μL of a negative reference standard from the reagent rack to two wells of the plate and adding 90 μL diluent. The positive reference standard and positive control are added from the reagent rack similarly. Blood, plasma or serum samples are then pipetted onto the microplate from the test tubes in the amount of 10 μL with 90 μL diluent. The contents of the wells are mixed and then incubated for 30 minutes at 37°C. The samples are then washed several times and the sample processor adds 100 μL of conjugate reagent to all wells. After mixing, they are incubated again for 15 minutes at 37°C. The wells are washed again and 200 μL of substrate reagent is added. The plate is then incubated at 37°C for 10-15 minutes and if the optical density of the positive reference standard is greater than 1.2A, then 50 μL of stopping reagent is added otherwise the plate is incubated longer. After waiting 5 minutes the optical densities of all the wells are read at 405 nm by the spectrophotometer. These values are then transmitted to the computer and the EIA percentages are calculated.

In order for the test results to be valid, a minimum of a 4:1 ratio between the optical densities of the positive and negative reference standards should be imposed. An acceptable range for the positive control is 30% to 85% enzyme immunoassay. Acceptable ranges of the optical densities of the negative and positive reference standards are 0.1 to 0.4 and 0.8 to 2.0 respectively. The optical densities of any duplicates should fall within 15% of their average. If any of the values fall outside of these desired predetermined ranges, then the program which displays this information will flash these values, alerting the user to the inconsistency. According to an important feature of the invention, any samples with an EIA percentage greater than a certain threshold value will be displayed with flashing or other enhanced video characteristics. The values of the optical densities and EIA percentages may alternatively be printed out on paper. This provides a worker with the results of the test, any positive results being clearly marked with the carcass label so as to assist the worker in identifying and locating the carcass in the slaughterhouse.

An example of another test which may be carried out using the method of the present invention is the detection of violative levels of sulfamethazine in swine. Such a detection would result from an antibiotic residue present in a sample taken from a carcass and is indicative of the abuse of an animal with antibiotics. The test is based on a standard ELISA and uses either of the alkaline phosphatase or horseradish peroxidase as the reporter enzyme. A plasma sample may be used which is uniquely identified and organized as in the previous example. Qualitative tests such as thin layer chromatography also exist for swine blood, serum and urine. Any of these may be used in accordance with the present invention. For such a test, the computer will be used to determine the level of sulfamethazine, with a level above 0.1 μg/ml typically being a positive identification of a violative level. Such a test is easily carried out in the manner according to the present invention as previously described and is effective in detecting abusive or illegal use of antibiotics in the animals. Similarly, other immmunoassay tests may be used in accordance with the present invention in order to determine other characteristics of interest from the carcasses.

As can be seen from the foregoing detailed description, the present invention provides an efficient and automated method for testing carcasses entering a slaughterhouse for harmful or infectious matter. Of primary importance to this testing method is its capability to produce results quickly from test samples taken from each carcass, such that any positive results are known well before the dismemberment and processing of the carcass to which the positive sample relates. Further in this regard, individual test samples are uniquely related to the carcass from which they were taken. When a particular sample produces a positive test result, this unique relation allows the related carcass to be identified such that it may then be located in the slaughterhouse and removed from the routine processing procedure.

## Claims

1. A method for preventing the routine processing in a slaughterhouse of carcasses which contain infected edible mammalian tissue, said method comprising the steps of:

(a) collecting a non-solid tissue sample from each carcass to be processed:

(b) uniquely coding each of said carcasses and providing said corresponding samples with corresponding codes

(c) testing each of said samples for a characteristic associated with infected or contaminated edible mammalian tissue. wherein the results of said testing are available within said finite time frame for each respective sample: and

(d) upon the results of said test for a particular sample indicating the presence of said characteristic, using said code associated with said particular sample for locating and preventing the routine processing and distribution of the carcass designated by the corresponding code of said particular sample.

2. The method according to Claim 1, further comprising a step (e) performed after steps (a) and (b) of routing said carcasses for said routine high-speed processing in said slaughterhouse.

3. The method according to Claim 1, wherein said non-solid tissue samples are blood samples, plasma samples, serum samples, urine samples, or lymph-fluid samples.

4. The method according to Claim 2 or Claim 3, wherein said testing is an enzyme immunoassay.

5. The method according to any one of the preceding claims, wherein a plurality of said non-solid tissue samples are collected from a plurality of carcasses and said plurality of samples are grouped together for substantially simultaneous testing, wherein said results of said testing are displayed on a display screen or printed onto a hardcopy printout.

6. The method according to Claim 5, wherein said plurality of samples of a group are arranged in a predetermined organized manner and the test results of said group are displayed on a display screen with an organization substantially similar to the organization of the samples of said group.

7. A method for detecting the presence of certain matter in a carcass in a high-speed slaughterhouse operation, said method comprising the steps of:

uniquely coding each of said carcasses and collecting a non-solid tissue sample from each of said carcasses upon said carcasses entering said slaughterhouse operation;

uniquely coding each of said non-solid tissue samples from said carcasses;

entering and recording said non-solid tissue sample codes and said carcass codes into a computer so that each sample code is uniquely related to its associated carcass code;

preparing one or more assay samples from each of said non-solid tissue samples;

determining the analytical value of each of said assay samples;

reading the analytical value of each of said assay samples into said computer and relating said analytical values to one or more control values to produce a test result value for each assay sample;

comparing each of said test result values to a predetermined range of values related to said certain matter; and

upon a test result value having a predetermined relationship to said predetermined range of values, determining the unique carcass code associated with said test result value having said predetermined relationship to said predetermined range of values.

8. The method according to Claim 7, wherein a plurality of different assay samples are prepared for detecting the presence of a respective plurality of different types of certain matter.

9. The method according to Claim 7, wherein said non-solid tissue samples are blood samples or lymph-fluid samples and said analytical values are optical density values.

10. The method according to Claim 9, wherein a plurality of said non-solid tissue samples are collected from a plurality of carcasses and said plurality of samples are grouped together wherein the optical densities of all the samples in each said group of samples are determined substantially simultaneously, wherein said test result values are displayed on a display screen or printed onto a hardcopy printout.

9

FIG.1

EP 0 295 349 A2

FIG.2

FIG.3

FIG.4

FIG. 5

```
┌─────────────────────────────────────────────────────────────┐
│                  SAMPLE ID ENTRY STATION                      │
└─────────────────────────────────────────────────────────────┘
```

DATE:  4/15/87       TIME:  12:31    — 56

OPERATOR ID:         55 →             50 → JOHN C. JONES

LOT INFO:                             51 → 118665 RC 2-3/4

TEST ID:                              52 → TRICHINELLA

PLATE/RACK FORMAT                     53 → DUPLICATE

PLATE/RACK ID:       57 ↴             54 → A0321

```
┌─────────────────────────────────────────────────────────────┐
│                     INPUT INFORMATION                         │
│      USE KEYBOARD TO ENTER YOUR NAME OR EMPLOYEE NUMBER       │
│                                                               │
│                                                               │
│                                                               │
│                                                               │
└─────────────────────────────────────────────────────────────┘
```

## FIG.6

| | 1 | 2 | 3 | 4 | 5 | 6 | 7 | 8 | 9 | 10 | 11 | 12 |
|---|---|---|---|---|---|---|---|---|---|---|---|---|
| A | NRS | NRS | 019 | 019 | | | | | | | | |
| B | PRS | PRS | 020 | 020 | | | | | | | | |
| C | PC | PC | 021 | 021 | | | | | | | | |
| D | 014 | 014 | | | | | | | | | | |
| E | 015 | 015 | | | | | | | | | | |
| F | 016 | 016 | | | | | | | | | | |
| G | 017 | 017 | | | | | | | | | | |
| H | 018 | 018 | | | | | | | | | | |

61

60

SAMPLE:  9                          PLATE FORMAT:  DUPLICATE
LOCATION:  D3, D4                    PLATE/RACK ID:  A0321

## FIG. 7

BEGIN

BAR CODE
TEST TUBE — 68

LABEL CARCASS — 69

COLLECT FLUID SAMPLE IN
BAR CODED TEST TUBE — 70

PLACE BAR CODED TEST TUBE WITH
FLUID SAMPLE IN TEST TUBE RACK — 71

BAR CODE TEST TUBE
RACK WITH MICROPLATE CODE — 72

ENTER PLATE FORMAT
INTO COMPUTER — 73

PLACE SAMPLES IN WELLS
OF PRE-COATED MICROPLATES — 74

PREPARATION OF
ASSAY — 75

DETERMINE OPTICAL
DENSITIES — 76

READ OPTICAL
DENSITIES INTO COMPUTER — 77

CALCULATE PERCENTAGE
ENZYME IMMUNOASSAY — 78

DETERMINE CARCASS LABELS FOR
EIA PERCENTAGES ABOVE THRESHOLD — 79

END

FIG.8

## OPTICAL DENSITY VALUES

| | 1 | 2 | 3 | 4 | 5 | 6 | 7 | 8 | 9 | 10 | 11 | 12 |
|---|---|---|---|---|---|---|---|---|---|---|---|---|
| A | NRS 0.228 | NRS 0.211 | 8186 0.205 | 8186 0.174 | 8194 0.170 | 8194 0.175 | 8202 0.145 | 8202 0.139 | 8210 0.207 | 8210 0.211 | 8218 0.184 | 8218 0.163 |
| B | PRS 1.502 | PRS 1.444 | 8187 0.167 | 8187 0.159 | 8195 0.446 | 8195 0.447 | 8203 0.145 | 8203 0.138 | 8211 0.136 | 8211 0.137 | 8219 0.159 | 8219 0.157 |
| C | PC 1.069 | PC 1.032 | 8188 0.141 | 8188 0.143 | 8196 0.130 | 8196 0.129 | 8204 0.148 | 8204 0.153 | 8212 0.150 | 8212 0.147 | 8220 0.154 | 8220 0.167 |
| D | 8181 0.185 | 8181 0.185 | 8189 0.127 | 8189 0.122 | 8197 0.117 | 8197 0.119 | 8205 0.148 | 8205 0.144 | 8213 0.125 | 8213 0.126 | 8221 0.163 | 8221 0.167 |
| E | 8182 0.154 | 8182 0.152 | 8190 0.190 | 8190 0.178 | 8198 0.196 | 8198 0.201 | 8206 0.155 | 8206 0.148 | 8214 0.231 | 8214 0.232 | 8222 0.130 | 8222 0.141 |
| F | 8183 0.157 | 8183 0.159 | 8191 0.138 | 8191 0.135 | 8199 0.149 | 8199 0.152 | 8207 0.147 | 8207 0.145 | 8215 0.154 | 8215 0.156 | 8223 0.179 | 8223 0.172 |
| G | 8184 0.148 | 8184 0.137 | 8192 0.148 | 8192 0.147 | 8200 0.127 | 8200 0.134 | 8208 0.140 | 8208 0.138 | 8216 0.153 | 8216 0.151 | 8224 0.150 | 8224 0.166 |
| H | 8185 0.094 | 8185 0.091 | 8193 0.124 | 8193 0.123 | 8201 0.169 | 8201 0.167 | 8209 0.178 | 8209 0.179 | 8217 0.132 | 8217 0.133 | 8225 0.142 | 8225 0.142 |

## FIG. 9

## PERCENTAGE ENZYME IMMUNO ASSAY

| | 1 | 2 | 3 | 4 | 5 | 6 | 7 | 8 | 9 | 10 | 11 | 12 |
|---|---|---|---|---|---|---|---|---|---|---|---|---|
| A | NRS 0.229 | NRS 0.211 | 8186 -1 | 8186 -4 | 8194 -4 | 8194 -4 | 8202 -6 | 8202 -6 | 8210 -1 | 8210 -1 | 8218 -3 | 8218 -5 |
| B | PRS 1.502 | PRS 1.444 | 8187 -4 | 8187 -5 | 8195 19 | 8195 18 | 8203 -6 | 8203 -7 | 8211 -7 | 8211 -7 | 8219 -5 | 8219 -5 |
| C | PC 68 | PC 65 | 8188 -6 | 8188 -6 | 8196 -7 | 8196 -7 | 8204 -6 | 8204 -5 | 8212 -6 | 8212 -6 | 8220 -5 | 8220 -4 |
| D | 8181 -3 | 8181 -3 | 8189 -7 | 8189 -8 | 8197 -8 | 8197 -8 | 8205 -6 | 8205 -6 | 8213 -8 | 8213 -7 | 8221 -5 | 8221 -4 |
| E | 8182 -5 | 8182 -5 | 8190 -3 | 8190 -3 | 8198 -2 | 8198 -1 | 8206 -5 | 8206 -6 | 8214 1 | 8214 1 | 8222 -7 | 8222 -6 |
| F | 8183 -5 | 8183 -5 | 8191 -7 | 8191 -7 | 8199 -6 | 8199 -5 | 8207 -6 | 8207 -6 | 8215 -5 | 8215 -5 | 8223 -3 | 8223 -4 |
| G | 8184 -6 | 8184 -7 | 8192 -6 | 8192 -6 | 8200 -7 | 8200 -7 | 8208 -6 | 8208 -7 | 8216 -5 | 8216 -5 | 8224 -6 | 8224 -4 |
| H | 8185 -10 | 8185 -10 | 8193 -8 | 8193 -8 | 8201 -4 | 8201 -4 | 8209 -3 | 8209 -3 | 8217 -7 | 8217 -7 | 8225 -6 | 8225 -6 |

## FIG. 10